# EUROPEAN PATENT APPLICATION

(11) **EP 3 449 808 A1**
(43) Date of publication of application: **06.03.2019**
(21) Application number: 17460056.9
(22) Date of filing: 05.09.2017
(51) Int. Cl.: A61B 3/032, A61B 3/024

(54) **VISUAL FIELD TESTING DEVICE WITH PATIENT REFRACTIVE ERROR MEASUREMENT AND CORRECTION**

(71) Applicant: Frey Spolka Jawna, 05-502 Piaseczno (PL)
(72) Inventor: Frey, Wojciech, 02-972 Warsaw (PL); Frey, Jacek, 05-502 Piaseczno (PL)

(57) **Abstract**

Visual field testing device with function of measuring and correcting patient refractive error is presented. One embodiment of the device according to the invention involves DLP technology for optotype and stimulus generation and a electrically tunable lens for refractive error correction. Alternative designs for measuring and providing refractive error correction are described. The refractive error can be measured and corrected manually or automatically with the device.

## Description

### Classifications

A61B3/024 Subjective types, i.e. testing apparatus requiring the active assistance of the patient for determining the visual field, e.g. perimeter types

### TECHNICAL FIELD

Various embodiments of the present invention relate generally to the field of visual field testing. In particular, the invention is directed towards an improved means of measuring and correcting the refractive error in a perimeter or other visual field testing device, to increase the reliability of the visual field test, improve the ease of use of the instrument, and improve patient comfort.

### BACKGROUND OF THE INVENTION

Requirements for visual field testing systems are standardized by ISO 12866 and many perimeters conforming to the standard requirements are available on the market. During visual field test patient's perception to test stimuli over a range of locations is analyzed while the patient's gaze is fixed on a single location. Usually for gaze tracking video camera embedded into projection surface is used, patient response is recorded with use of pushbutton.

For measuring refractive error usually patient is seated in front of Phoroptor or is wearing trial lens frames and is asked to read chart of optotypes, it is letters, numbers, pictures or special signs of different size, while eye professional is changing optical power of lenses. The procedure continues until patient is able to read required size optotype. Methods of refractive error measurement and requirements for test charts are well described in international standards ISO 8596, ISO 8597, ISO 10938 and in the literature, for example "Primary Care Optometry" 5 edition by Theodore Grosvenor, published by Elsevier Inc. ISBN 978-0-7575-8.

Correction of patient's refractive error is important to achieve a reliable result on a visual field test because refractive blur will reduce the visual sensitivity to perimetric stimuli. It is a standard practice today to reduce the refractive error to within 1 diopter with the use of a set of standard ophthalmic trial lenses. It is possible to correct spherical and non-spherical refractive error with standard trial lenses. The perimeter operator selects and inserts lenses into the line of vision of the patient based on previously determined values or according to information provided by the patient. A large fraction of all patients require such correction due to myopia, hyperopia or presbyopia. Trial lenses are available in most clinical settings, are commonly used method of reducing refractive blur during a visual field test. Mistakes in selecting the correct trial lens can cause a general depression of the visual field.

Other methods and systems for correction of refraction errors for visual field testing devices are presented in patent application US 2013/0070204 A1, however system according to this application does not provide reference test charts allowing to measure patient refractive error objectively. It is important to notice that refractive error changes in time, so the values of earlier measurements may not be accurate.

It is therefore a general objective of this invention to provide both, stimulus and optotype presentation system and method, and an visual field test apparatus with functionality allowing to measure patient refraction error, to improve the reliability of the visual field test, improve patient comfort, and reduce errors and possible mistakes associated with lack of information about correct value of patient refraction error.

### BRIEF SUMMARY OF THE INVENTION

The invention includes visual field testing apparatus, e.g., a perimeter, which measures a patient's response to a stimulus presented in the patient's visual field and the perimeter stimulus generation system capable of displaying stimulus and optotypes of variable shape and size and a system for correcting a patient's refractive error. The system includes optotype and stimulus presenting system and refractive correction optics used in conjunction with a perimeter system. The optotype of variable size and shape is generated and projected by stimulus projection system and refractive power of the correction lens is manually or automatically adjustable in a manner to correct a patient's refractive error and provide the patient with a sharp image of a visual stimulus within the tested area of the patient's visual field.

### BRIEF DESCRIPTION OF THE DRAWINGS

FIG. 1 shows a block diagram of one embodiment of the present invention.
FIG. 2 displays an embodiment of the stimuli and optotype projection system.
FIG. 3 illustrates refraction error measurement mode of operation of one embodiment of the present invention.

### DETAILED DESCRIPTION OF THE INVENTION

As described above, correction of patient refraction error is important for quality of perimetry examination results. Usually refractive error value is measured in separate examination and should be repeated as these values have tendency to change in time. The invention described herein presents visual field testing instrument capable of measuring and correcting a patient's refractive error.

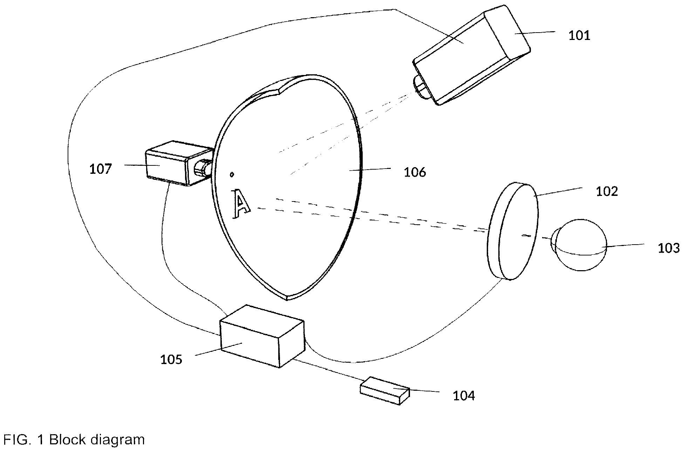

In a preferred embodiment of direct projection visual field testing device according to this invention, the same projection system is used for test stimulus and optotype projection and a refractive correction optics is attached to the front of the perimeter in either a fixed or removable fashion, but it could also be built into the perimeter itself. In either case, the lens device or frame providing the refractive correction could be removed when the central region of the visual field is not being tested, to allow for testing in other regions of the visual field. In the most basic sense, the invention incorporates optotype projecting system integrated with test stimulus projecting system and a lens device with a means to allow manual or automatic change of its refractive power, combined with a visual field testing device as shown in FIG. 1.

The instrument presented on FIG. 1 consists of a stimulus and optotype generation system **101** which projects test stimuli or optotype onto display surface **106** (in this case a bowl shaped), a camera **107** for collecting information on the position and gaze of the eye (e.g. vertex location, pupil location and gaze direction), a refractive correction optics **102** positioned in between the eye of a patient **103** and the display surface of the perimeter **106,** and a response detection system **104** for measuring the patient's perception of the various stimuli. A processor **105** interfaces with the device for analyzing the test results, controlling test stimuli and optotype shape and size, controlling the variable refractive correction optics in the case of automated adjustment, and for interfacing with the camera among other functions. The display surface **106** of the perimeter could be curved or flat, and the stimuli and optotype could be displayed in any number of arrangements including forward projection, back projection, or direct illumination of light sources (LEDs, OLEDs, etc). The response detection system **104** could be a button pressed by a patient or a means for recording an audible response of the patient among others.

A variety of different embodiments of the stimulus and optotype generation system can be imagined. Below we describe embodiment using DLP technology. It will be obvious to one skilled in the art to envision others that would fall into the scope of this invention. Many embodiments of variable refractive correction system are possible including electrically focus tunable lenses, liquid lenses, others. Operation of vision testing device according to this invention will be described using electrically focus tunable lens system.

The optotype presentation and refractive correction system could also be applied to other types of perimeters or other devices capable of measuring visual field or visual function.

### System with stimulus and optotype presentation system based on DLP technology and refraction error correction system based on electrically tunable lens.

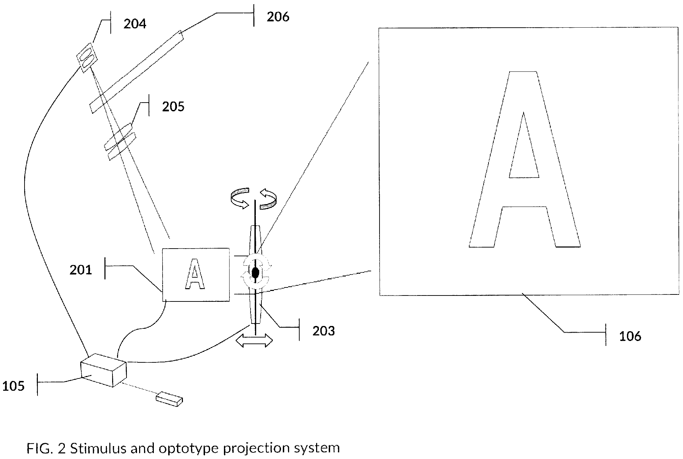

In preferred embodiment of the present invention, which is presented on **FIG 2****,** the stimulus and optotype generation system is realized around DLP integrated circuit **201** which combines plurality of tiltable mirrors arranged in matrix of columns and rows. DLP IC is equipped with circuits to control tilt angle of every mirror of the DLP matrix and this way is able to control angle of light reflection from DLP surface.DLP IC is illuminated by light source **204** and optical system **205.** Optional filter wheel **206** can be used for stimulus color modification or alternatively variable color light source can be used. Processor **105** controls operation of DLP IC to generate required shape of stimuli and the optotype. Light reflected from DLP towards projection system **203** will form image on display surface **106.** Opto-mechanical projection system **203** is used to direct beam of light reflected from DLP onto certain part of the display surface **106** and to control focus of the image projected. Electrically tunable lenses can be used in construction of projection system **203.**

The optotype and stimulus projection system according to this invention can work in two modes, optotype presentation for measurement of patient refractive error and in stimulus projection mode for visual field testing mode. In optotype presentation mode different size letters, digits, special characters, contrast sensitivity patterns or entire lines of text can be generated on DLP IC and then projected onto the display surface. In stimulus projection mode usually circle of required size is generated by DLP and projected by projection system into required position. In stimulus projection mode DLP IC can also be used to correct shape of the stimuli generated when projection surface is not perpendicular to projection system to assure always circular shape of the stimuli. Different shapes and animated sequences of stimulus can be generated using DLP IC to allow implementation of various visual field test techniques. Optotype and stimulus projection system operation mode and image generated is controlled by processor **105** which can also control stimulus or optotype projection position and focus.

Refraction correction system in preferred embodiment of the visual field testing device according to this invention utilizes electrically tunable lens which can change its refractive power according to applied electric current or voltage. Good example of such lens is commercially available product from Optotune www.optotune.com or Holochip https://www.holochip.com/. Refraction correction system works in two modes, in refraction error measurement mode and visual field testing mode. Refractive power of the correction system in both modes is controlled by processor **105.**

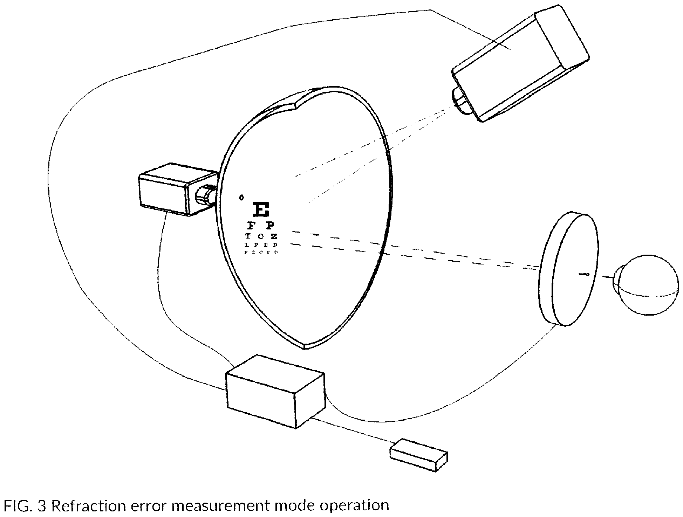

Patient refraction measurement is first stage of patient examination and allows for determining real value of refraction error of the patient. During this stage projection system of the visual field testing device works in optotype projection mode and projects set of optotypes of variable size onto projection surface as presented on **FIG 3****.** Patient observing, through the correction lens, optotypes projected onto test surface is asked by device operator to name/read displayed character lines. Operator of the device can modify refractive value of correction lens until patient is able to read required size characters. This way patient refraction correction value is determined and first stage of the examination completed.

After first stage is completed device can start visual field test by switching projection system to stimulus projection mode and refractive power of refraction correction lens is set to value determined in first stage of the examination. The entire visual field test procedure continues until it is completed and results are stored in device memory or are printed.

Alternative embodiments of visual field testing device according to this invention are possible. This may include stimuli and optotype projection system build with LCD module instead of DLP IC. Another possible embodiment may use active type OLED, LCD, projection surface for presenting stimuli for visual field testing and optotypes for measuring refractive error.

Refraction error correction system can be realized with standard set of trial lenses and proper lens holder attached to the device, for manual correction of the refraction error. In manual correction configuration of the device, stimulus and optotype projection system is set to optotype projection mode and the operator of the device inserts trial lenses of various refractive values into the lens holder until patient is able to read required size optotype.

## Claims

1. An apparatus for analyzing the visual field of a patient, said apparatus comprising:
a display surface;
a visual stimulus system that generates stimuli at various locations on the display surface;
an optotype generation system combined into stimulus generation system;
a response detection system for collecting data on the patient's perception of the visual stimuli; and
a variable refractive correction optics attached to said apparatus for correcting the refractive error of the patient.

2. An apparatus as recited in claim 1, wherein the stimulus and optotype generation system include a DLP integrated circuit.

3. An apparatus as recited in claim 1, wherein the optotype generation system is capable of displaying different size and shape graphical signs and characters, letters or digits.

4. An apparatus as recited in claim 1, wherein the optotype generation system is capable of displaying different contrast graphical signs and characters, letters or digits.

5. An apparatus as recited in claim 1, wherein the stimulus generation system is capable of displaying animated stimuli.

6. An apparatus as recited in claim 1, wherein the variable refractive correction optics include electrically tunable lens.

7. An apparatus as recited in claim 1, wherein the variable refractive correction optics include a pair of aligned rotatable wheels, each wheel carrying a plurality of lenses having different diopter values.

8. An apparatus as recited in claim 1, wherein the refractive power of the variable refractive correction optics is adjusted manually.

9. An apparatus as recited in claim 1, wherein the refractive power of the variable refractive correction optics is adjusted automatically based on the refractive error value of the patient measured with the apparatus.

10. An apparatus as recited in claim 1, wherein the variable refractive correction optics provide spherical and cylindrical refractive correction.

11. An apparatus as recited in claim 1, further comprising one or more auxiliary lenses to extend the range of the refractive correction.

12. An apparatus for analyzing the visual field of a patient, said apparatus comprising:
an active display surface capable of displaying optotypes;
a visual stimulus system that generates stimuli at various locations on the display surface;
a response detection system for collecting data on the patient's perception of the visual stimuli; and
variable refractive correction optics operable attached to said apparatus for correcting the refractive error of the patient.

13. An apparatus as recited in claim 12, wherein the display surface include OLED display.

14. An apparatus as recited in claim 12, wherein the display surface include LCD display.

15. An apparatus for analyzing the visual field of a patient, said apparatus comprising:
an active display surface for displaying optotypes and visual stimuli at various locations on the display surface;
a response detection system for collecting data on the patient's perception of the visual stimuli; and
variable refractive correction optics operable attached to said apparatus for correcting the refractive error of the patient.

16. An apparatus as recited in claim 15, wherein the display surface include OLED display.

17. An apparatus as recited in claim 15, wherein the display surface include LCD display.
